# EUROPEAN PATENT APPLICATION

(11) **EP 2 051 080 A1**
(43) Date of publication of application: **22.04.2009**
(21) Application number: 07807873.0
(22) Date of filing: 26.09.2007
(51) Int. Cl.: G01N 33/70

(54) **D/P CREATININE MARKER, METHOD OF DETERMINING D/P CREATININE AND USE OF THE SAME**

(30) Priority: 28.09.2006 JP 2006266032
(71) Applicant: JMS Co., Ltd., Hiroshima-shi, Hiroshima 730-8652 (JP)
(72) Inventor: HIRAI, Satoshi, Hiroshima 730-8652 (JP); KARINO, Tomokazu, Hiroshima 730-8652 (JP)
(74) Representative: Woods, Geoffrey Corlett
(86) International application number: PCT/JP2007/068658
(87) International publication number: WO 2008/038655

(57) **Abstract**

A method of indirectly determining D/P creatinine for easily judging a peritoneal function is provided that does not require blood sampling or multiple recoveries of dialysis effluent. The method includes preparing a dialysis effluent obtained by peritoneal dialysis, measuring at least one protein marker selected from the group consisting of prealbumin, haptoglobin, alpha 1-microglobulin, C4, and total protein that are contained in the dialysis effluent, and determining D/P creatinine indirectly from the correlation equation that represents the relationship between the marker concentration and the D/P creatinine value that has been prepared beforehand. Furthermore, the PET category is determined indirectly from the D/P creatinine, which has been determined indirectly, based on the known reference value.

## Description

### Technical Field

This invention relates to a marker to serve as an index of D/P creatinine that is used as a reference for a peritoneal function, a method of indirectly determining D/P creatinine, and an intended use thereof.

### Background Art

It is known that when peritoneal dialysis, a therapy for renal failure, is performed for a long period of time, the stimulus thereof tends to decrease the peritoneal function of the patient. This causes peritoneal permeability to be aggravated, which results in a decrease in the dialysis effect and a risk of onset of encapsulating peritoneal sclerosis (EPS).

Therefore, in the field of dialysis therapy, the decrease in peritoneal function in dialysis patients needs to be diagnosed at an early stage. Currently, a peritoneal equilibration test (PET) is used as a method of testing the peritoneal function (see Nonpatent Document 1). The PET is a method of evaluating the peritoneal function in four levels (four categories). In this method, generally, a dialysate is accumulated in the peritoneum of a patient, and the creatinine concentration and glucose concentration in the dialysate and blood are measured over time. Thereafter, the water removal ability and waste product removal ability of the peritoneum are evaluated from the changes in creatine concentration and glucose concentration during the dialysis period. With the evaluation results, the peritoneal function is classified into a category that indicates the level thereof. The change in creatinine concentration serves as an index of the waste product removal ability, and the change in glucose concentration as an index of the water removal ability. Specifically, when the peritoneal function is classified according to the change in creatinine concentration, the value of "D/P creatinine" is used. This value denotes the ratio between a creatinine concentration (D) in a dialysate (dialysis effluent) and a creatinine concentration (P) in blood at an arbitrary time during dialysis therapy. The relationship between the D/P creatinine value and each category employed in the PET is disclosed in, for example, Nonpatent Document 2. Classification falls into four categories, "high", "high average", "low average", and "low", sequentially from higher D/P creatinine values, i.e. higher permeability. Therefore, classification into the four categories performed according to the D/P creatinine values measured from dialysis patients makes it possible to determine, for example, adjustment of dialysate osmotic pressure, frequency of dialysis, use in combination with hemodialysis, and transition to hemodialysis according to the results of the classification. Thus, the classification into the categories by the PET is used to prevent complications as described above from developing. With respect to the four categories employed in the PET, more particularly, the category "high", which denotes high permeability, indicates relatively that the waste product removal ability is high and the water removal ability is low; "high average", which denotes slightly higher permeability, indicates relatively that the waste product removal ability is average and the water removal ability is slightly lower; "low average", which denotes slightly lower permeability, indicates relatively that the waste product removal ability is slightly lower and the water removal ability is average; and "low", which denotes low permeability, indicates relatively that the waste product removal ability is low and the water removal ability is high.

However, in order to perform classification into PET categories by measuring D/P creatinine as described above, the dialysate needs to be drained after the start of dialysis several times (for example, zero hour, two hours, and four hours after the start of the dialysis). Additionally, blood sampling also is required. This constrains patients to recover effluent for a certain period of time and is also accompanied by the pain of blood sampling. Furthermore, there is a problem in that due to the difficulty of sampling at home, patients must go to a hospital, and therefore effort is required by both the patient and doctor. Furthermore, even in the case of using the fast PET method, which is a simplified version of the original PET method, the period of time for which patients are constrained to recover dialysate remains unchanged, and it is necessary to measure the creatinine concentrations of a total of four samples including blood and at least three types of dialysis effluents drained over time. Thus the measurement itself takes time and energy.
[Nonpatent Document 1] Twardowski ZJ, et al. Peritoneal equilibration test. Perit Dial Bull 1987; 7: 138-47.
[Nonpatent Document 2] "Yokuwakaru Fukumaku Touseki no Kiso┘, TOKYOIGAKUSHA, Akihiro YAMASHITA, issued October 1, 1998

### Disclosure of Invention

### Problem to be Solved by the Invention

Accordingly, the present invention is intended to provide a new marker that does not require blood sampling and multiple recoveries of dialysis effluent and can assess the peritoneal function easily.

### Means for Solving the Problem

A marker of the present invention is a marker for D/P creatinine (hereinafter referred to as a "D/P creatinine marker") and contains at least one protein selected from the group consisting of prealbumin, haptoglobin, alpha 1-microglobulin, C4, and total protein.

A method of determining D/P creatinine according to the present invention includes the following steps (a) to (c):
(a) preparing a peritoneal dialysis effluent collected from a subject,
(b) measuring a D/P creatinine marker of the present invention that is contained in the effluent, and
(c) calculating the value of the D/P creatinine from the value of the marker measured in step (b) based on a correlation equation that indicates the relationship between the value of the D/P creatinine and the amount of the D/P creatinine marker.

A method of judging a PET category of the present invention includes the following steps (A) and (B):
(A) determining D/P creatinine by a method of determining D/P creatinine of the present invention, and
(B) judging the PET category based on the D/P creatinine thus determined.

As a result of keen studies made assiduously, the inventors found out that the content of a certain group of proteins varied in a peritoneal dialysis effluent, depending on the peritoneal function (peritoneal permeability) of a peritoneal dialysis patient. That is, the contents of a total of five types of proteins, namely, prealbumin, haptoglobin, alpha 1-microglobulin, and total protein, varied between the peritoneal dialysis effluent of a patient having high peritoneal permeability and that of a patient having low peritoneal permeability. As a result of further studies, the inventors found out the correlation between the D/P creatinine value and the amount of the proteins in the peritoneal dialysis effluent. Therefore, as in the present invention, using those proteins as D/P creatinine markers, the amount of them contained in the peritoneal dialysis effluent is measured, and based on the result, the D/P creatinine of the peritoneal dialysis patient can be determined indirectly. Furthermore, based on the result thus determined, classification into the PET categories also can be performed. In the conventional PET, both the dialysis effluent and blood need to be collected as samples as mentioned above. According to the method of the present invention, however, it is enough just to collect the dialysis effluent, and thereby the pain of the patient also can be reduced. Moreover, according to the method of the present invention, one dialysis effluent is sufficient as the sample to be used for testing the peritoneal function. Therefore multiple recoveries of effluent are not required and it is sufficient to measure one dialysis effluent. Accordingly, the operation itself is considerably simple as compared to the conventional PET. Particularly, the conventional PET is conducted usually approximately once a year due to the complication of the process thereof and a heavy burden on the patient and doctor. On the other hand, since the method of the present invention is very simple, it can be conducted frequently. Therefore, when the D/P creatinine and PET category are determined using the marker of the present invention as a target, less effort is required by both the patient and doctor and the peritoneal function can be tested easily. Since it can be conducted frequently, the transition between the PET categories can be detected at an early stage. This also makes it possible to detect, at an early stage, the transitional period where the PET category changes from "high average" to "high", which is considered to be particularly important. Thus, the present invention is very useful, for example, for changing the dialysis conditions according to peritoneal symptoms and preventing the onset of peritonitis due to long-term peritoneal dialysis in the treatment of renal failure.

### Brief Description of Drawings

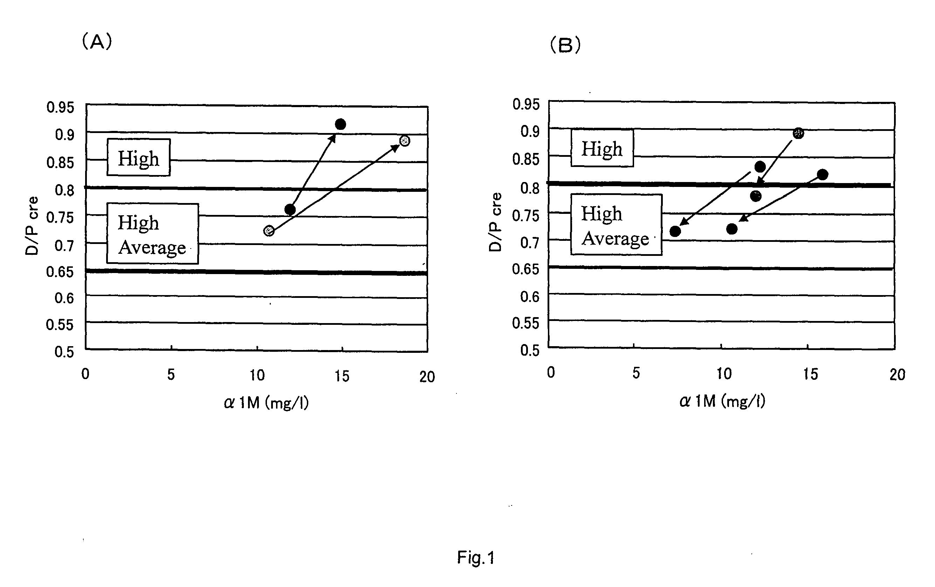

FIG. 1 shows graphs illustrating the correlations between the change in true D/P creatinine and the change in alpha 1-microglobulin in the same patient; (A) shows the result obtained from a patient with the category being changed from high average to high, and (B) shows the result obtained from a patient with the category being changed from high to high average.

### Best Mode for Carrying Out the Invention

### <D/P creatinine marker>

As described above, the D/P creatinine marker of the present invention contains at least one protein selected from the group consisting of prealbumin, haptoglobin, alpha 1-microglobulin, C4, and total protein.

All these proteins serve as markers that indicate a relative increase in concentration of D/P creatinine, that is, a relative increase in peritoneal permeability, with a relative increase in concentration of the proteins in peritoneal dialysis effluents. Specifically, in the case of a dialysis effluent with a relatively high D/P creatinine value (i.e. relatively high permeability), for example, the dialysis effluent has a higher marker concentration as compared to an effluent with a relatively low D/P creatinine value (i.e. relatively low permeability). Accordingly, as described later, with the measurement of the markers in dialysis effluents, when the concentrations or amounts thereof are relatively high, it can be judged that the D/P creatinine value is relatively high and thus the peritoneal permeability is high. The specific judgment method is described later. Among those markers, alpha 1-microglobulin, prealbumin, and total protein are preferable, and alpha 1-microglobulin is particularly preferable because it shows the highest correlation with the D/P creatinine.

### <Method of determining D/P creatinine>

Next, a method of determining D/P creatinine of the present invention is characterized by including the following steps (a) to (c) as described above:
(a) preparing a peritoneal dialysis effluent collected from a subject,
(b) measuring a D/P creatinine marker of the present invention that is contained in the effluent, and
(c) calculating the value of the D/P creatinine from the value of the marker measured in step (b) based on a correlation equation that indicates the relationship between the value of the D/P creatinine and the amount of the D/P creatinine marker.

An example of the method of determining D/P creatinine of the present invention is described below, but the present invention is not limited thereto.

First, the dialysate accumulated in the peritoneum of a patient is drained. The duration of the accumulation is not particularly limited. However, it is preferable that the same condition be employed, for example, among patients and for each analysis. Specifically, an analysis sample is, for example, a dialysate drained after accumulation in a peritoneal cavity for 8 to 12 hours.

Subsequently, with respect to the analysis sample thus obtained, the D/P creatinine marker of the present invention is measured (qualitatively or quantitatively). In the present invention, any one of prealbumin, haptoglobin, alpha 1-microglobulin, C4, and total protein can be measured as the D/P creatinine marker. The method of measuring the marker is not particularly limited. A conventionally known detection method for measuring proteins can be employed. Furthermore, since the aforementioned proteins themselves are known proteins, it also is possible to employ the conventionally known methods for detecting the respective proteins. Even though those proteins themselves are known, it was found by the inventors for the first time that they each serve as a marker for D/P creatinine and the D/P creatinine can be determined indirectly by measuring the amount or concentration thereof in a peritoneal dialysis effluent.

Specific examples of the aforementioned detection method include an enzymatic method, an immunologic technique, and electrophoresis. The immunologic technique can be a conventionally known method that utilizes an antigen-antibody reaction. For example, the following can be employed: enzyme-linked immuno sorbent assay (ELISA method), Western blotting, turbidimetric immunoassay such as latex agglutination turbidimetric immunoassay, colloidal gold agglutination assay, and immunonephelometry (nephelometry). The antibodies to be used for the methods may be, for example, commercially available antibodies or may be monoclonal antibodies or polyclonal antibodies that are prepared independently.

The marker that is measured by the immunologic technique can be detected further easily with a test device that utilizes a conventionally known technique. An example of the test device for measuring the D/P creatinine marker by the ELISA method is a test piece with a reagent for the marker that is placed on a substrate having a porous structure or capillary structure. Examples of the reagent include an antibody to the D/P creatinine marker and an antigen (marker). In the case of a test piece with an anti-D/P creatinine antibody immobilized as the reagent, first, peritoneal dialysis effluent is allowed to drip and thereby an antigen reaction occurs between the immobilized antibody and the antigen (D/P creatinine marker) contained in the peritoneal dialysis effluent. Further, a labeled anti-D/P creatinine antibody is added thereto to undergo an antigen-antibody reaction with the D/P creatinine marker bound to the immobilized antibody. The label of the labeled antibody bound to the D/P creatinine marker then is detected. Thus the D/P creatinine marker contained in the effluent can be measured. Moreover, with a labeled D/P creatinine marker and peritoneal dialysis effluent containing a D/P creatinine marker being added to a test piece with an anti-D/P creatinine antibody immobilized thereto, an unlabeled marker and a labeled marker may be bound competitively to the antibody. In this case, the detection of the label of the labeled marker bound to the immobilized antibody allows the antigen D/P creatinine marker contained in the effluent to be measured.

The aforementioned substrate is not limited. Preferably, it can support (for instance, immobilize) the reagent and is formed of a material (for instance, a material with a high water absorptivity) or structure that is capable of developing the peritoneal effluent. Examples of the substrate include filter paper, chromatography paper, and nonwoven fabric, as well as a polymer porous body. The material for the porous body is not limited. Examples thereof include cellulose polymers such as cellulose, cellulose acetate, and nitrocellulose, polyethylene, polypropylene, and polyamide resins (for instance, nylon), resins formed using polyester or acrylonitrile as a raw material, and other resins such as polytetrafluoroethylene. Furthermore, when the test piece is used, a conventionally known method can be employed suitably for improving, for example, measurement sensitivity, measurement precision, and operability of a series of processes.

Examples of the electrophoresis include one-dimensional electrophoresis and two-dimensional electrophoresis. These techniques make it possible to separate all the markers by, for example, single electrophoresis (one-dimensional and two-dimensional electrophoreses). Therefore, it also is possible to measure all the markers at once based on the presence or absence of spots of the markers in the gel after electrophoresis or the color tone of the spots. Preferably, the same electrophoresis conditions and amount of whole protein in a sample to be used for the electrophoresis are employed for all the samples. Staining is not particularly limited. Examples thereof include silver staining, Coomassie Brilliant Blue (CBB) staining, and fluorescent staining such as SYPRO Ruby staining (trade name).

Prealbumin, haptoglobin, alpha 1-microglobulin, C4, and total protein are known proteins as described above, and each of them is known as a blood biochemical test item. Accordingly, the concentration or amount in the peritoneal dialysis effluent may be measured by utilizing a known measurement method. Generally, the method of measuring prealbumin is, for example, turbidimetry, the method of measuring haptoglobin or C4 is, for example, turbidimetric immunoassay, the method of measuring alpha 1-microglobulin is, for example, latex agglutination immunoassay, and the method of measuring total protein is, for example, the Biuret method.

For example, the D/P creatinine can be calculated indirectly from the measured value based on the correlation equation that represents the correlation between the D/P creatinine marker and the D/P creatinine. Hereinafter, the D/P creatinine that is calculated indirectly according to the present invention is referred to as "estimated D/P creatinine", and the D/P creatinine that is calculated directly from its concentration in blood and its concentration in dialysis effluent is referred to as "true D/P creatinine". This correlation equation can be made by, for example, measuring the true D/P creatinine of a plurality of dialysis patients by a conventional method beforehand while measuring the D/P creatinine markers in the peritoneal dialysis effluents of the aforementioned dialysis patients and then analyzing both the measurement results. Thereafter, the measured value of the D/P creatinine marker in the dialysis effluent of each subject is assigned to the correlation equation. Thus the estimated D/P creatinine can be determined indirectly. In making the correlation equation, the period of time for collecting the dialysis effluent (the dialysate accumulation time) to be used for measuring the true D/P creatinine is not particularly limited. Preferably, however, it is constant among the plurality of dialysis effluents to be measured and is, for example, two to four hours, preferably four hours. On the other hand, the period of time for collecting dialysis effluent (the dialysate accumulation time) to be used for measuring the D/P creatinine marker (for calculating the estimated D/P creatinine) is not particularly limited. It is, for example, 6 to 14 hours, preferably 8 to 12 hours, and more preferably 8 hours. Preferably, the same also applies to the period of time for collecting dialysis effluent of a subject.

The correlations between the D/P creatinine markers of the present invention and the true D/P creatinine measured using blood and the peritoneal dialysis effluent recovered after four hour accumulation are shown below as specific examples. The present invention, however, is not limited to these correlation equations.

**[Table 1]**

| | Correlation with D/P creatinine |
|---|---|
| alpha 1-microglobulin | y = 0.0246x + 0.4845 |
| | Correlation Coefficient: 0.673 |
| prealbumin | y = 0.1188x + 0.5486 |
| | Correlation Coefficient: 0.515 |
| haptoglobin | y = 0.0369x + 0.6422 |
| C4 | y = 0.2696x + 0.5971 |
| total protein | y = 0.0013x + 0.5089 |
| | Correlation Coefficient: 0.559 |

| | |
|---|---|
| y: D/P creatinine x: Marker concentration alpha 1-microglobulin: "mg/L" others: "mg/dL" | |

As described above, when the peritoneal dialysis is continued, the peritoneal function decreases. Consequently, the D/P creatinine increases, and the aforementioned PET category shifts toward "high". It has been reported that the patient categorized into "high" has a high risk of onset of, for example, peritonitis or sclerosing encapsulating peritonitis. In order to prevent the onset of such peritonitis, it is necessary to study, for example, the peritoneal rest period, the timing for discontinuation of dialysis, and the combined use of hemodialysis or transition to hemodialysis, in the peritoneal dialysis treatment. It has been known that generally, when the PET category is "high average", the stage preceding "high", the best condition is obtained and good peritoneal dialysis can be performed. Therefore, detecting the transitional period from the "high average" to "high", particularly, detecting the increasing behavior exhibited in the range of numerical values of D/P creatinine that are categorized into "high average" is considered to be very important. However, as described before, the conventional PET requires blood sampling and multiple dialysate recoveries at set recovery times and therefore is accompanied by patient's pain and great effort is required by both the patient and doctor. Therefore a frequent periodical measurement was not performed. According to the present invention, however, blood sampling is not required and only a single recovery of peritoneal dialysis effluent is necessary. Thus it is possible to recover samples frequently and periodically. Furthermore, this is not limited to the samples recovered in the hospital, and it is sufficient to send the samples recovered at home to the hospital or testing institution by mail. This can reduce the effort required by both the patient and doctor. Furthermore, it also becomes possible for patients to measure the marker of the present invention at home using, for example, a measurement kit or a simple measuring device to check the estimated D/P creatinine.

### <Method of judging PET category>

As described above, the method of judging the PET category of the present invention is characterized by including the following steps (A) and (B):
(A) determining D/P creatinine by a method of determining D/P creatinine of the present invention, and
(B) judging the PET category based on the D/P creatinine thus determined.

In the PET that currently is employed in peritoneal function tests, the peritoneal function is classified into four categories according to the range in which the D/P creatinine value falls. As described above, the D/P creatinine marker of the present invention in a peritoneal dialysis effluent has a correlation with the D/P creatinine. Accordingly, when the estimated D/P creatinine is determined indirectly through the measurement of the D/P creatinine marker as described above, based on the result, it can be classified indirectly into a PET category.

The correlation equations as mentioned above can be used for determining the estimated D/P creatinine. In making each correlation equation, when a sample obtained by, for example, being drained four hours after the start of dialysis is used for the measurement of the true D/P creatinine, the estimated D/P creatinine in the effluent obtained four hours after the start of dialysis can be calculated from the correlation equation. In this case, for example, the estimated D/P creatinine in the range of approximately 0.81 to 1.03 is classified into a category of "high", that in the range of approximately 0.65 to 0.81 into "high average", that in the range of approximately 0.5 to 0.65 into "low average", and that in the range of approximately 0.34 to 0.5 into "low". When the estimated D/P creatinine value is, for example, a border value between respective categories, it is not necessary to determine strictly into which category it falls. For instance, even in the case of a value that belongs to "high average", it can be judged that the risk to become "high" or the possibility to be "high" is high as it approaches the upper limit thereof. Therefore, for example, when the estimated D/P creatinine is 0.81, it can be judged to be "high" or it can be judged to be "high average" with a significant risk of becoming "high". These are mere examples and the present invention is not limited thereto.

Examples of the method of indirectly judging the PET category by detecting the D/P creatinine marker of the present invention include not only the method of indirectly determining the estimated D/P creatinine as described above but also, for example, the following methods. The present invention is not limited to these embodiments at all.

### <First Embodiment>

The dialysis effluent of a subject is subjected to electrophoresis (for example, one-dimensional or two-dimensional). The gel subjected to the electrophoresis is stained, and the spot of the D/P creatinine marker in the electrophoresis gel thus stained is observed. In this case, it can be judged that a relatively darker spot of the D/P creatinine marker according to the present invention indicates a patient having high permeability, i.e. a patient having a relatively high D/P creatinine value with the category thereof being relatively on the "high" side.

### <Second Embodiment>

With respect to the dialysis effluent of a dialysis patient whose PET category has been known, it is subjected to electrophoresis (for example, one-dimensional or two-dimensional) and staining of the electrophoresis gel beforehand to be employed as an evaluation reference. This electrophoretic profile is used as an evaluation reference. For the evaluation reference, respective effluents of patients in four PET categories may be used, or among them, an effluent of a patient in a PET category into which classification is intended may be used. Specific examples include a combination of categories "high" and "high average", and a combination of categories "high" and "low" or "low average". On the other hand, the dialysis effluent of a subject is subjected to electrophoresis and gel staining under the same conditions. Subsequently, the electrophoretic profile of the subject and the electrophoretic profile of the evaluation reference are compared with respect to the presence or absence of spots of the D/P creatinine marker or the color tone of stained spots. The category into which the evaluation reference, in which the D/P creatinine marker exhibits the similar behavior, falls can be estimated as the PET category of the subject.

### <Third Embodiment>

With respect to the dialysis effluent of a dialysis patient whose PET category has been known, the content of each marker per a certain amount of whole protein is measured beforehand to be employed as an evaluation reference. The content of each marker in each category is employed as the evaluation reference. For the evaluation reference, respective effluents of patients in four PET categories may be used, or among them, an effluent of a patient in a PET category into which classification is intended may be used. Specific examples include a combination of categories "high" and "high average", and a combination of categories "high" and "low" or "low average". On the other hand, with respect to the dialysis effluent of a subject, the content of each marker per a certain amount of total protein is measured in the same manner. Subsequently, the content of each marker of the subject is compared with the content of the evaluation reference. The category into which the evaluation reference having a comparable content falls can be estimated as the category of the subject. This method is suitable when prealbumin, haptoglobin, alpha 1-microglobulin, or C4 is employed as the D/P creatinine marker.

### <Fourth Embodiment>

With respect to the dialysis effluent of a dialysis patient whose PET category has been known, the concentration of a D/P creatinine marker is measured beforehand to be employed as an evaluation reference. For the evaluation reference, respective effluents of patients in four PET categories may be used, or among them, an effluent of a patient in a PET category into which classification is intended may be used. Specific examples include a combination of categories "high" and "high average", and a combination of categories "high" and "low" or "low average". On the other hand, with respect to the dialysis effluent of a subject, the concentration of the D/P creatinine marker is measured in the same manner. The method of measuring the D/P creatinine marker concentration is not particularly limited. For example, conventionally known protein detection methods can be used including, for example, a method in which the antigen-antibody reaction with the anti-marker antibody is used and a method using HPLC. The marker concentration to be detected thus measured is compared to the evaluation reference. Thus the category into which the evaluation reference having a comparable concentration falls can be estimated as the category of the subject.

Furthermore, using a commercially available computer, it also is possible to determine the correlation between estimated D/P creatinine and true D/P creatinine as well as the estimated D/P creatinine, the PET category, or the peritoneal function based on the correlation between the estimated D/P creatinine and the PET category, and further to determine the future dialysis schedule. Specifically, a system can be employed in which from the measured value of the D/P creatinine marker of the dialysis effluent of a subject, the estimated D/P creatinine is calculated based on the aforementioned correlation equations, the estimated D/P creatinine thus obtained is compared to the known reference value of the true D/P creatinine whose PET category is to be judged, and thus the PET category is judged. Further, for example, a data processor containing a program for executing such a series of steps in a computer and a recording medium that can be read by a computer with the program stored therein can be used. Moreover, examples of the general indices for evaluation of the peritoneal function of a patient include the concentration of each solute and the amount of water that has been removed. There is a known method of testing the peritoneal function such as the elute removal ability or the water removal ability by using the Pyle-Popovich model, which is known as a macroscopic pharmacokinetic modeling of peritoneal dialysis, based on the data described above. Accordingly, a combination of this method of testing the peritoneal function and the method of the present invention allows the peritoneal function to be evaluated in further detail.

Hereinafter, the present invention is described in further detail using examples but is not limited thereto.

### [Example 1]

It was checked whether the D/P creatinine marker of the present invention had a correlation with the true D/P creatinine according to the conventional method.

With respect to 33 patients, for the first measurement, using the peritoneal dialysate accumulated for four hours, the true D/P creatinine was measured by a conventional method and concentrations of various D/P creatinine markers were measured by the method described later. Furthermore, for the second measurement, with respect to 13 patients out of the aforementioned 33 patients, the true D/P creatinine and the concentrations of various D/P creatinine markers were measured again 6 to 12 months after the first measurement. For the third measurement, with respect to three patients out of the aforementioned 13 patients, the true D/P creatinine and the concentrations of various D/P creatinine markers were measured in the same manner 6 to 12 months after the second measurement. Further, for the fourth measurement, one patient out of the aforementioned three patients, the true D/P creatinine and the concentrations of various D/P creatinine markers were measured in the same manner 6 to 12 months after the third measurement. The concentrations of D/P creatinine markers were measured by the following method using, as samples, peritoneal dialysis effluents, each of which was obtained by accumulating dialysate in the peritoneal cavity of each patient for 8 to 12 hours and then draining it.

### <Measurement of prealbumin concentration>

With respect to 30 µL of sample, prealbumin was measured by immunonephelometry (nephelometry) using N-antiserum prealbumin (Dade Behring Co., Ltd.).

### <Measurement of haptoglobin concentration>

With respect to 30 µL of sample, haptoglobin was measured by immunonephelometry (nephelometry) using N-antiserum haptoglobin (Dade Behring Co., Ltd.).

### < Measurement of alpha 1-microglobulin concentration>

With respect to 8 µL of sample, alpha 1-microglobulin was measured by turbidimetric immunoassay (latex agglutination turbidimetric immunoassay) using LX reagent 'Eiken' α1-M-III (trade name, Eiken Chemical Co., Ltd.).

### < Measurement of C4 concentration>

With respect to 5 µL of sample, C4 was measured by turbidimetric immunoassay (latex agglutination turbidimetric immunoassay) using N-Assay TIA C4-SH Nittobo (trade name, Nitto Boseki Co., Ltd.).

### < Measurement of total protein concentration>

With respect to 350 µL of sample, total protein was measured by the Biuret method.

From a total of 50 results of the measurements of concentrations of true D/P creatinine and various markers, correlation equations were determined, each of which represents the correlation between the true D/P creatinine and each marker. The results are indicated in the table below. As indicated in the table below, the true D/P creatinine and each marker in the peritoneal dialysate accumulated for four hours had a correlation therebetween. Particularly, the alpha 1-microglobulin had a very high correlation with the true D/P creatinine. From the above-mentioned results, it became clear that it was possible to estimate the D/P creatinine indirectly through the measurement of the D/P creatinine marker in a peritoneal dialysis effluent and further to judge the PET category thereof indirectly based on the estimated D/P creatinine. Furthermore, 33 patients were classified into categories by the conventional PET as follows: in the first measurement, five patients were "high", 16 patients "high average", nine patients "low average", and three patients "low"; and in the second measurement carried out with respect to 13 patients out of the 33 patients, four patients were "high", six patients "high average", two patients "low average", and one patient "low". Thus, the aforementioned correlations are considered to be reliable in the ranges of all of the low to high categories.

**[Table 2]**

| | Correlation with D/P creatinine |
|---|---|
| alpha 1-microglobulin | y = 0.0246x + 0.4845 |
| | Correlation Coefficient: 0.673 |
| prealbumin | y = 0.1188x + 0.5486 |
| | Correlation Coefficient: 0.515 |
| haptoglobin | y = 0.0369x + 0.6422 |
| C4 | y = 0.2696x + 0.5971 |
| total protein | y = 0.0013x + 0.5089 |
| | Correlation Coefficient: 0.559 |

| | |
|---|---|
| y: D/P creatinine x: Marker concentration alpha 1-microglobulin: "mg/L" others: "mg/dL" | |

### [Example 2]

In Example 1, with respect to five patients out of the 13 patients subjected to the second measurement carried out 6 to 12 months after the first measurement, their PET categories determined from the true D/P creatinine were different from those determined in the first measurement. Therefore, with respect to each of those patients, it was checked whether the alpha 1-microglobulin concentration varied with the change in true D/P creatinine.

Based on the measurement results of Example 1, the graphs shown in FIG. 1 indicate the relationship between the change in true D/P creatinine and the change in alpha 1-microglobulin (α1M) concentration in the same patient whose PET category varied. FIG. 1(A) shows the results of transition of the PET category from high average to high, and FIG. 1(B) shows the results of transition of the PET category from high to high average. In both the graphs, the arrows each indicate the direction of category transition with respect to the same patient, and the bold lines in the graphs indicate D/P creatinine values showing sequentially from the top the borderline between high and high average and the borderline between high average and low average.

As shown in FIG. 1, it was proved that the alpha 1-microglobulin concentration increased with an increase in true D/P creatinine that serves as a reference for the classification into PET categories, while the alpha 1-microglobulin concentration decreased with a decrease in true D/P creatinine.

### <Reference Example 1>

It was checked whether alpha 1-microglobulin in an effluent and the remaining renal function had a correlation therebetween.

### (1) Correlation with remaining renal function

With respect to 14 patients, each of which had a remaining renal function (with a urine volume of at least 100 mL), the alpha 1-microglobulin concentration in each effluent was measured in the same manner as in Example 2. Furthermore, the creatinine clearance (how much fluid is filtered by the kidneys in one minute) of each of the same patients was checked by a conventional method. Thereafter, the correlation between the alpha 1-microglobulin concentration in the effluent and the urine volume of the patient as well as the correlation between the alpha 1-microglobulin concentration in the effluent and the creatinine clearance were checked. As a result, the alpha 1-microglobulin concentration in the effluent had no correlation with both the urine volume and the creatinine clearance. In other words, the alpha 1-microglobulin concentration in the effluent had no correlation with the remaining renal function of the patient.

### (2) Correlation between alpha 1-microglobulin and D/P creatinine of patient classified according to remaining renal function

Effluent samples recovered in the same manner as in Example 2 were classified into a group of samples obtained from patients with remaining renal functions (with a urine volume of at least 100 mL, 36 patients) and a group of samples obtained from patients with no remaining renal function (with a urine volume of less than 100 mL, 14 patients). The alpha 1-microglobulin concentration in each sample was measured. With respect to these patients, the D/P creatinine was measured by the conventional method in the same manner as in Example 2. Thereafter, with respect to each of the patients with remaining renal function and the patients with no remaining renal function, the correlation between the alpha 1-microglobulin and D/P creatinine was checked. As a result, regardless of the presence or absence of the remaining renal function, the correlation between the alpha 1-microglobulin and D/P creatinine was found as indicated below.

**[Table 3]**

| Remaining renal function | Correlation between D/P creatinine and alpha 1-microglobulin |
|---|---|
| Present (n = 36) | y = 0.0286x + 0.4445 |
| | Correlation Coefficient: 0.6571 |
| Absence (n = 14) | y = 0.0202x + 0.5333 |
| | Correlation Coefficient: 0.795 |

| | |
|---|---|
| y: D/P creatinine x: alpha 1-microglobulin (mg/L) | |

As mentioned in item (1) above, the alpha 1-microglobulin in the effluent and the remaining renal function had no correlation therebetween. Furthermore, as mentioned in item (2) above, the alpha 1-microglobulin in the effluent had a correlation with the D/P creatinine, based on which the PET category was determined, regardless of the presence or absence of the remaining renal function. Accordingly, it is considered that the analysis method that utilizes alpha 1-microglobulin in the dialysis effluent as a marker allows the D/P creatinine and PET category of every peritoneal dialysis patient to be estimated-without being affected by the presence or absence of the remaining renal function of the patient.

### Industrial Applicability

As described above, the measurement of the amount of the marker according to the present invention in a peritoneal dialysis effluent allows the D/P creatinine of the patient to be determined indirectly and further classification into a PET category to be performed based on the result thus determined. Furthermore, since unlike the conventional PET, it is sufficient to collect only a dialysis effluent, patient's pain can be reduced and the test is simplified. Particularly, the conventional PET generally is carried out only once a year due to the complicated process thereof and the heavy burden on the patient and doctor. As described above, however, the method of the present invention is very simple and therefore can be carried out frequently. Accordingly, when the D/P creatinine and the PET judging method are used with the marker of the present invention to serve as a target, less effort is required by both the patient and doctor and the peritoneal function can be tested easily. Particularly, since it is possible to detect, at an early stage, the transitional period where the PET category changes from "high average" to "high", the present invention is very useful, for example, for preventing the onset of peritonitis that is caused by long-term peritoneal dialysis due to the change in the treatment method.

## Claims

1. A marker for D/P creatinine, comprising at least one protein selected from the group consisting of prealbumin, haptoglobin, alpha 1-microglobulin, C4, and total protein.

2. The marker according to claim 1, wherein the marker indicates a relative increase in D/P creatinine with a relative increase in amount of protein.

3. The marker according to claim 1, wherein the marker is alpha 1-microglobulin.

4. The marker according to claim 1, wherein the protein is a protein contained in a peritoneal dialysis effluent.

5. A method of determining D/P creatinine, wherein the method comprising:
(a) preparing a peritoneal dialysis effluent collected from a subject,
(b) measuring a marker for D/P creatinine according to claim 1 that is contained in the effluent, and
(c) calculating the value of the D/P creatinine from the value of the marker measured in process (b) based on a correlation equation that indicates the relationship between the value of the D/P creatinine and the amount of the marker for the D/P creatinine.

6. The method of determining D/P creatinine according to claim 5, wherein the correlation equation has been made from D/P creatinine values obtained from a plurality of dialysis patients and measurement values of D/P creatinine markers obtained from the plurality of dialysis patients.

7. The method of determining D/P creatinine according to claim 6, wherein the D/P creatinine values obtained from the plurality of dialysis patients are those obtained from effluents collected two to four hours after the start of peritoneal dialysis.

8. The method of determining D/P creatinine according to claim 6, wherein the measurement values of D/P creatinine markers obtained from the plurality of dialysis patients are those obtained from effluents collected 8 to 12 hours after the start of peritoneal dialysis.

9. The method of determining D/P creatinine according to claim 8, wherein in process (a), the peritoneal dialysis effluent collected from a subject is an effluent collected 8 to 12 hours after the start of peritoneal dialysis.

10. The method of determining D/P creatinine according to claim 5, wherein a method of measuring the marker is a method that utilizes at least one of an immunologic technique and electrophoresis.

11. A method of judging a PET category, comprising:
(A) determining D/P creatinine by a method of determining D/P creatinine according to claim 5, and
(B) judging the PET category based on the D/P creatinine thus determined.

12. The method of determining a PET category according to claim 11, wherein the PET category is discriminated between a high average category and a high category.
